(19) Europäisches Patentamt / European Patent Office / Office européen des brevets

(11) **EP 3 840 872 B1**

(12) **EUROPÄISCHE PATENTSCHRIFT**

(45) Veröffentlichungstag und Bekanntmachung des Hinweises auf die Patenterteilung:
**30.08.2023   Patentblatt 2023/35**

(21) Anmeldenummer: **19749380.2**

(22) Anmeldetag: **09.08.2019**

(51) Internationale Patentklassifikation (IPC):
***B01J 13/00*** *(2006.01)*

(52) Gemeinsame Patentklassifikation (CPC):
**B01J 13/0065; B01J 13/0069**

(86) Internationale Anmeldenummer:
**PCT/EP2019/071450**

(87) Internationale Veröffentlichungsnummer:
**WO 2020/038742 (27.02.2020 Gazette 2020/09)**

(54) **VERFAHREN ZUR HERSTELLUNG VON SUPERABSORBERN**

METHOD FOR THE PRODUCTION OF SUPERABSORBENTS

PROCÉDÉ DE FABRICATION DE SUPERABSORBANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.08.2018   EP 18189715**

(43) Veröffentlichungstag der Anmeldung:
**30.06.2021   Patentblatt 2021/26**

(73) Patentinhaber: **BASF SE**
**67056 Ludwigshafen am Rhein (DE)**

(72) Erfinder:
• **PFEIFFER, Thomas**
  **67056 Ludwigshafen (DE)**
• **FUNK, Ruediger**
  **67056 Ludwigshafen (DE)**

• **KRUEGER, Marco**
  **67056 Ludwigshafen (DE)**
• **POSSEMIERS, Karl**
  **2040 Antwerpen (BE)**
• **SCHROEDER, Juergen**
  **67056 Ludwigshafen (DE)**
• **WEISMANTEL, Matthias**
  **67056 Ludwigshafen (DE)**

(74) Vertreter: **BASF IP Association**
**BASF SE**
**GBI-C006**
**67056 Ludwigshafen (DE)**

(56) Entgegenhaltungen:
| | |
|---|---|
| **EP-A1- 2 371 869** | **WO-A1-2010/040466** |
| **WO-A1-2010/040467** | **WO-A1-2013/120722** |
| **US-A1- 2010 099 799** | **US-A1- 2010 105 808** |

**Beschreibung**

Die vorliegende Erfindung betrifft ein Verfahren zur Herstellung von Superabsorberpartikeln durch Polymerisation einer Monomerlösung, umfassend die Schritte kontinuierliche Polymerisation der Monomerlösung auf dem umlaufenden Band eines Bandreaktors, Zerkleinerung des erhaltenen Polymergels, Spülen des auf der Unterseite des Bandreaktors anfallenden Polymergels mit Wasser in eine Abtrenneinheit, Abtrennung des Polymergels vom Wasser in der Abtrenneinheit und Rückführung des Polymergels in die Zerkleinerung, wobei das Polymergel eine Quellkapazität in Wasser von mindestens 100 g/g aufweist, die Verweilzeit des Polymergels im Spülwasser weniger als 20 Minuten beträgt und die Abtrenneinheit Öffnungen mit einer lichten Weite von mindestens 1 mm aufweist.

Superabsorber werden zur Herstellung von Windeln, Tampons, Damenbinden und anderen Hygieneartikeln, aber auch als wasserzurückhaltende Mittel im landwirtschaftlichen Gartenbau verwendet. Die Superabsorber werden auch als wasserabsorbierende Polymere bezeichnet.

Die Herstellung von Superabsorbern wird in der Monographie "Modern Superabsorbent Polymer Technology", F.L. Buchholz und A.T. Graham, Wiley-VCH, 1998, Seiten 71 bis 103, beschrieben.

Die Eigenschaften der Superabsorber können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm$^2$ (AUL0.3psi) durchläuft ein Maximum.

Zur Verbesserung der Anwendungseigenschaften, wie beispielsweise Gelbettpermeabilität (GBP) und Absorption unter einem Druck von 49,2 g/cm$^2$ (AUL0.7psi), werden Superabsorberpartikel im allgemeinen oberflächennachvernetzt. Dadurch steigt der Vernetzungsgrad der Partikeloberfläche, wodurch die Absorption unter einem Druck von 49,2 g/cm$^2$ (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächen nachvernetzt. Dazu geeignete Vernetzer sind Verbindungen, die mit mindestens zwei Carboxylatgruppen der Polymerpartikel kovalente Bindungen bilden können.

Die Herstellung von Superabsorbern auf dem umlaufenden Band eines Bandreaktors wird beispielsweise in DE 38 25 366 A1 und US 6,241,928 beschrieben. EP 2 371 869 A1 offenbart ein Verfahren zur Herstellung von Superabsorberpartikel durch Polymerisation einer Monomerlösung umfassend die Rückführung von Feinanteilen.

Aufgabe der vorliegenden Erfindung war die Bereitstellung eines verbesserten Verfahrens zur Herstellung von Superabsorbern durch Polymerisation auf dem umlaufenden Band eines Bandreaktors, insbesondere eine verbesserte Rückführung des auf der Unterseite des Bandreaktors anfallenden Polymergels.

Gelöst wurde die Aufgabe durch ein Verfahren zur Herstellung von Superabsorbern durch Polymerisation einer Monomerlösung, enthaltend

a) mindestens ein ethylenisch ungesättigtes, säuregruppentragendes Monomer, das zu 50 bis 85 mol-% neutralisiert ist,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

umfassend die Schritte

i) kontinuierliche Polymerisation der Monomerlösung auf dem umlaufenden Band eines Bandreaktors,
ii) Zerkleinerung des in Schritt i) erhaltenen Polymergels,
iii) Spülen des auf der Unterseite des Bandreaktors anfallenden Polymergels mit Wasser in eine Abtrenneinheit,
iv) Abtrennung des Polymergels vom Wasser in der Abtrenneinheit und
v) Rückführung des in Schritt iv) abgetrennten Polymergels in Schritt ii),

wobei das Polymergel eine Quellkapazität in Wasser von mindestens 100 g/g aufweist, die Verweilzeit zwischen Schritt iii) und Schritt iv) weniger als 20 Minuten beträgt und die Abtrenneinheit in Schritt iv) Öffnungen mit einer lichten Weite von mindestens 1 mm aufweist.

Die Verweilzeit zwischen Schritt iii) und Schritt iv) beträgt vorzugsweise weniger als 15 Minuten, besonders bevorzugt weniger als 10 Minuten, ganz besonders bevorzugt weniger als 5 Minuten.

Die Verweilzeit ist dabei die Zeit zwischen dem ersten Kontakt eines Polymergelpartikels mit dem Spülwasser und Eintritt des Polymergelpartikels in die Abtrenneinheit. Das System sollte Totraumfrei sein, d.h. bei Abschalten des Spülwassers sollte das System zwischen Zufuhr des Spülwassers und Ablauf der Abtrenneinheit leerlaufen. In diesem Fall kann die Verweilzeit bestimmt werden indem eine konstante Menge an Spülwasser zugeführt wird bis ein stationärer Zustand erreicht wird und anschließend die Zufuhr an Spülwasser gestoppt wird. Die Menge an Spülwasser, die nach der Abschaltung der Zufuhr an Spülwasser aus der Abtrenneinheit abläuft, wird gemessen. Die Verweilzeit kann dann gemäß der folgenden Formel berechnet werden:

$$VWZ\,[min] = x[m^3]/y[\frac{m^3}{min}]$$

VWZ     Verweilzeit
x         nach Abschaltung der Zufuhr ablaufendes Spülwasser
y         konstante Zufuhr an Spülwasser

Die lichte Weite der Öffnungen ist der Abstand gegenüberliegender Seiten der Öffnung. Eine lichte Weite von mindestens 1 mm bedeutet, dass dieser Wert nirgends unterschritten wird. Die lichte Weite der Öffnungen beträgt vorzugsweise mindestens 1,5 mm, besonders bevorzugt mindestens 2 mm, ganz besonders bevorzugt mindestens 3 mm. Die lichte Weite sollte 15 mm nicht überschreiten.

Die Zerkleinerung kann mit jedem geeignetem Apparat durchgeführt werden, beispielsweise mit einem Extruder.

Wasser im Sinne der vorliegenden Erfindung ist Leitungswasser, demineralisiertes Wasser und/oder aus dem Verfahren rückgeführtes Wasser, insbesondere Spülwasser.

Der vorliegenden Erfindung liegt die Erkenntnis zugrunde, dass die Polymergelpartikel in dem Spülwasser stark quellen, dadurch leichter deformierbar sind und die Öffnungen der Abtrenneinheit leichter passieren. Dieses unerwünschte Quellen kann durch kurze Verweilzeiten deutlich vermindert werden. Außerdem lassen sich die weniger gequollenen Polymergelpartikel anschließend leichter trocknen.

In einer bevorzugten Ausführungsform der vorliegenden Erfindung wird das auf der Unterseite des Bandreaktors anfallende Polymergel in einer Wanne aufgefangen. Dabei kann an der Oberfläche des rücklaufenden Bandes haftendes Polymergel allein durch die Schwerkraft oder mechanisch gelöst werden. Im Falle eines mechanischen Ablösens kann dies durch einen Wasserstrahl und/oder Bürsten erfolgen.

Die Abtrenneinheit weist Öffnungen zur Abtrennung des Wassers auf. Geeignete Öffnungen sind die Maschen eines Siebes und/oder Schlitze.

Die Maschen des Siebes weisen eine Maschenweite von mindestens 1 mm, vorzugsweise mindestens 1,5 mm, besonders bevorzugt mindestens 2 mm, ganz besonders bevorzugt mindestens 3 mm, auf. Die Maschenweite sollte 15 mm nicht überschreiten.

Die Schlitze weisen eine Breite von mindestens 1 mm, vorzugsweise mindestens 1,5 mm, besonders bevorzugt mindestens 2 mm, ganz besonders bevorzugt mindestens 3 mm, auf. Die Breite der Schlitze sollte 15 mm nicht überschreiten. Die Läge der Schlitze unterliegt keiner Beschränkung und liegt beispielsweise im Bereich von 20 bis 100 mm. Zu lange Schlitze sind nachteilig für die mechanische Stabilität der Abtrenneinheit.

In einer bevorzugten Ausführungsform der vorliegenden Erfindung ist die Abtrenneinheit eine liegende, rotierende Trommel und die Trommel ist vom Eingang zum Ausgang geneigt. Dabei wird das Wasser mit dem Polymergel am oberen Ende der Trommel zugeführt. Das Wasser kann durch die Öffnungen in der Trommelwand ablaufen und das Polymergel verlässt die Trommel am unteren Ende. Das abgetrennte Polymergel wird in den Extruder nach der Polymerisation rückgeführt.

Die liegende, rotierende Trommel ist um vorzugsweise mindestens 10°, besonders bevorzugt mindestens 20°, ganz besonders bevorzugt mindestens 30°, gegenüber der Horizontalen geneigt.

Das in Schritt iv) erhaltene Wasser kann in das Verfahren in Schritt iii) rückgeführt werden.

Zusammen mit dem in Schritt v) rückgeführten Polymergel können auch im Verfahren abgetrennt zu kleine Polymerpartikel ("fines") rückgeführt oder weitere Additive zugesetzt werden.

Im Folgenden wird die Herstellung der Superabsorber näher erläutert:
Die Superabsorber werden durch Polymerisation einer Monomerlösung hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 02/055469 A1, der WO 03/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomers a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 03/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 0,8 Gew.-%, ganz besonders bevorzugt 0,15 bis 0,5 Gew.-%, jeweils berechnet auf die Gesamtmenge an eingesetztem Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird vorzugsweise das Dinatriumsalz der 2-Hydroxy-2-sulfonatoessigsäure oder ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit der Löslichkeit überschreitendem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Die Polymerisation auf dem Band wird beispielsweise in DE 38 25 366 A1 und US 6,241,928 beschrieben. Bei der Polymerisation in einem Bandreaktor entsteht ein Polymergel, das zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen. Feste Carbonate und Hydrogencarbonate können hierbei auch in verkapselter Form eingesetzt werden, vorzugsweise in die Monomerlösung direkt vor der Polymerisation, während oder nach der Polymerisation ins Polymergel und vor dessen Trocknung. Die Verkapselung erfolgt durch Beschichtung der Oberfläche mit einem unlöslichen oder nur langsam löslichen Material (beispielsweise mittels filmbildender Polymere, inerter anorganischer Materialien oder schmelzbarer organischer Materialien), welches die Lösung und Reaktion des festen Karbonats oder Hydrogenkarbonats so verzögert, dass erst während der Trocknung Kohlendioxid freigesetzt wird und der entstehende Superabsorber eine hohe innere Porosität aufweist.

Optional kann zur Monomerlösung vor der Polymerisation ein Tensid zugegeben werden und die Monomerlösung dann mit einem Inertgas oder Wasserdampf oder durch starkes Rühren geschäumt werden. Das Tensid kann anionisch, kationisch, zwitterionisch oder auch nicht-ionisch sein. Bevorzugt wird ein hautfreundliches Tensid eingesetzt.

Das Polymergel wird dann in üblicherweise mit einem Umluftbandtrockner getrocknet bis der Restfeuchtegehalt vorzugsweise 0,5 bis 10 Gew.-%, besonders bevorzugt 1 bis 6 Gew.-%, ganz besonders bevorzugt 1,5 bis 4 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Polymergels beträgt vor der Trocknung vorzugsweise von 25 und 90 Gew.-%, besonders bevorzugt von 35 bis 70 Gew.-%, ganz besonders bevorzugt von 40 bis 60 Gew.-%. Anschließend wird das getrocknete Polymergel gebrochen und optional grob zerkleinert.

Das getrocknete Polymergel wird hiernach üblicherweise gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise von 150 bis 850 $\mu$m, besonders bevorzugt von 250 bis 600 $\mu$m, ganz besonders von 300 bis 500 $\mu$m. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Polymerpartikeln mit einer Partikelgröße von größer 150 $\mu$m beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Gelbettpermeabilität (GBP). Daher sollte der Anteil zu kleiner Polymerpartikel ("fines") niedrig sein.

Zu kleine Polymerpartikel werden daher üblicherweise abgetrennt und in das Verfahren rückgeführt, vorzugsweise unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Der Anteil an Polymerpartikeln mit einer Partikelgröße von höchstens 850 µm, beträgt vorzugsweise mindestens 90 Gew.-%, besonders bevorzugt mindestens 95 Gew.-%, ganz besonders bevorzugt mindestens 98 Gew.-%.

Der Anteil an Polymerpartikeln mit einer Partikelgröße von höchstens 600 µm, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Quellgeschwindigkeit. Daher sollte der Anteil zu großer Polymerpartikel ebenfalls niedrig sein. Zu große Polymerpartikel werden daher üblicherweise abgetrennt und in die Mahlung rückgeführt.

Die Polymerpartikel können zur weiteren Verbesserung der Eigenschaften thermisch oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 03/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 3 Gew.-%, besonders bevorzugt 0,02 bis 1 Gew.-%, ganz besonders bevorzugt 0,05 bis 0,2 Gew.-%, jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächennachvernetzt.

Bevorzugte Reaktionstemperaturen liegen im Bereich 100 bis 250°C, bevorzugt 110 bis 220°C, besonders bevorzugt 120 bis 210°C, ganz besonders bevorzugt 130 bis 200°C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die Polymerpartikel nach der Oberflächennachvernetzung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die Polymerpartikel auf vorzugsweise 40 bis 90°C, besonders bevorzugt 45 bis 80°C, ganz besonders bevorzugt 50 bis 70°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 40 bis 120°C, besonders bevorzugt bei 50 bis 110°C, ganz besonders bevorzugt bei 60 bis 100°C, durchgeführt. Bei zu niedrigen Temperaturen neigen die Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Oberflächennachvernetzung durchgeführt.

Geeignete Beschichtungen zur Verbesserung der Quellgeschwindigkeit sowie der Gelbettpermeabilität (GBP) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20. Geeignete Beschichtungen zur Staubbindung, zur Verringerung der Verbackungsneigung sowie zur Erhöhung der mechanischen Stabilität sind Polymerdispersionen, wie in EP 0 703 265 B1 beschrieben, und Wachse, wie in US 5 840 321 beschrieben.

Anschließend können die beschichteten und/oder nachbefeuchteten Polymerpartikel erneut klassiert werden, wobei zu kleine und/oder zu große Polymerpartikel abgetrennt und in das Verfahren rückgeführt werden.

Ein weiterer Gegenstand der vorliegenden Offenbarung sind Hygieneartikel, die gemäß dem erfindungsgemäßen Verfahren hergestellte Superabsorber enthalten.

Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategie Partners" EDANA (Avenue Eugène Plasky, 157, 1030 Brüssel, Belgien, www.edana.org) und INDA (1100 Crescent Green, Suite 115, Cary, North Carolina 27518, USA, www.inda.org). Diese Veröffentlichung ist sowohl von EDANA als auch von INDA erhältlich.

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

Feststoffgehalt

Zur Bestimmung des Feststoffgehaltes (FG) wird das Polymergel für mindestens 3 Stunden bei 180°C im Trockenschrank getrocknet (bis zur Gewichtskonstanz). Der Feststoffgehalt ist das Gewicht des getrockneten Polymergels dividiert durch die Einwaage.

Quellkapazität in Wasser (Free Swell Capacity, De-Ionized Water)

Die Quellkapazität in Wasser ($FSC_{DIW}$) wird analog der von der EDANA empfohlenen Testmethode Nr. WSP 240.2-05 "Free Swell Capacity in Saline, Gravimetric Determination" bestimmt, wobei das Polymergel vor der Messung getrocknet, gemahlen und auf einen Partikelgrößenbereich von 150 µm bis 850 µm abgesiebt wird, und die Messung in demineralisiertem Wasser statt in einer 0,9 gew.-%igen Kochsalzlösung durchgeführt wird. Bei höheren Quellkapazitäten ist der Teebeutel entsprechend zu vergrößern.

Beispiele

Zur Simulation der kontinuierlichen Polymerisation auf einem Band wurde jeweils eine statische Polymerisation durchgeführt. Zur Simulation des Einbringens eines mit Polymergel beladenen Spülwassers in die Abtrenneinheit wurde das Polymergel auf ein grobmaschiges Sieb aufgebracht und mit Wasser nachgespült.

Beispiel 1

1028 g einer 37,3 gew.-%igen wässrigen Natriumacrylatlösung, 98 g Acrylsäure, 254 g Wasser und 0,30 g 3-fach ethoxyliertes Glyzerintriacrylat wurden in einem 2.000 ml Metallbecher eingewogen. Der Neutralisationsgrad betrug 75 mol-%. Der Metallbecher wurde mit Parafilm® verschlossen und mit 150 l/h Stickstoff inertisiert. Während des Inertisierens wurde die Monomerlösung auf 3°C gekühlt. Anschließend wurden nacheinander 6,47 g einer 10 gew.-%igen wässrigen Lösung von Natriumpersulfat und 5,88 g einer 1 gew.-%igen wässrigen Lösung von Wasserstoffperoxid zugegeben.

Die Monomerlösung wurde mittels eines Trichters in eine Glasschale mit einem Durchmesser von 190 mm überführt. Die Glasschale wurde mit einer Kunststofffolie abgedeckt und ebenfalls mit 150 l/h Stickstoff inertisiert. Zusätzlich wurde die Monomerlösung in der Glasschale mittels eines Magnetrührstabes gerührt. Anschließend wurden mittels einer Einwegspritze 5,88 g einer 1 gew.-%igen wässrigen Lösung von Brüggolite® FF6 (Dinatriumsalz der 2-Hydroxy- 2-sulfinatoessigsäure) in die Monomerlösung dosiert. Nach dem Reaktionsstart wurde der Magnetrührer abgeschaltet.

Nach einer Reaktionszeit von 30 Minuten wurde das Polymergel entnommen und mit einem Extruder mit einer Lochplatte (13 mm Lochdurchmesser) zerkleinert.

Das erhaltene Polymergel hatte einen Feststoffgehalt von 35,0 Gew.-%.

Ein Teil des Polymergels wurde eine Stunde bei 160°C im Umlufttrockenschrank getrocknet. Die Beladung der Bleche mit Polymergel betrug 0,19 g/cm$^2$. Anschließend wurde mit einem Walzenstuhl in vier Stufen mit Spaltbreiten von 5 mm, 1000 µm, 600 µm und 400 µm zerkleinert und auf eine Partikelgröße von 150 µm bis 850 µm abgesiebt. Von dem so erhaltenen Superabsorberpartikeln wurde die Quellkapazität ($FSC_{DIW}$) bestimmt, die einen Wert von 162 g/g ergab.

Beispiel 2

950 g einer 37,3 gew.-%igen wässrigen Natriumacrylatlösung, 146 g Acrylsäure, 335 g Wasser und 0,63 g 3-fach ethoxyliertes Glyzerintriacrylat wurden in einem 2.000 ml Metallbecher eingewogen. Der Neutralisationsgrad betrug 65 mol-%. Der Metallbecher wurde mit Parafilm® verschlossen und mit 150 l/h Stickstoff inertisiert. Während des Inertisierens wurde die Monomerlösung auf 3°C gekühlt. Anschließend wurden nacheinander 6,47 g einer 10 gew.-%igen wässrigen Lösung von Natriumpersulfat und 5,88 g einer 1 gew.-%igen wässrigen Lösung von Wasserstoffperoxid zugegeben.

Die Monomerlösung wurde mittels eines Trichters in eine Glasschale mit einem Durchmesser von 190 mm überführt. Die Glasschale wurde mit einer Kunststofffolie abgedeckt und ebenfalls mit 150 l/h Stickstoff inertisiert. Zusätzlich wurde die Monomerlösung in der Glasschale mittels eines Magnetrührstabes gerührt. Anschließend wurden mittels einer Einwegspritze 5,88 g einer 1 gew.-%igen wässrigen Lösung von Brüggolite® FF6 (Dinatriumsalz der 2-Hydroxy- 2-sulfinatoessigsäure) in die Monomerlösung dosiert. Nach dem Reaktionsstart wurde der Magnetrührer abgeschaltet.

Nach einer Reaktionszeit von 30 Minuten wurde das Polymergel entnommen und mit einem Extruder mit einer Lochplatte (13 mm Lochdurchmesser) zerkleinert.

Das erhaltene Polymergel hatte einen Feststoffgehalt von 35,5 Gew.-%.

Ein Teil des Polymergels wurde eine Stunde bei 160°C im Umlufttrockenschrank getrocknet. Die Beladung der Bleche mit Polymergel betrug 0,19 g/cm$^2$. Anschließend wurde mit einem Walzenstuhl in vier Stufen mit Spaltbreiten von 5 mm, 1000 µm, 600 µm und 400 µm zerkleinert und auf eine Partikelgröße von 150 µm bis 850 µm abgesiebt. Von dem so erhaltenen Superabsorberpartikeln wurde die Quellkapazität ($FSC_{DIW}$) bestimmt, die einen Wert von 195 g/g ergab.

Beispiel 3

Für jeden Versuch wurde die eingesetzte Menge nassen Polymergels zuvor gewogen und der Feststoffgehalt (FG) des Polymergels bestimmt.

Jeweils ca. 20 g Polymergel wurden nicht vorgequollen (0 Minuten Quellzeit) bzw. jeweils ca. 10 g Polymergel für 3 Minuten (3 Minuten Quellzeit), 30 Minuten (30 Minuten Quellzeit) bzw. 60 Minuten (60 Minuten Quellzeit) in 2 l deionisiertem Wasser vorgequollen.

Anschließend wurden die vorgequollenen Polymergele zusammen mit dem Wasser aus den verwendeten Polyethylenbechern jeweils auf eine Siebkombination aus einem oberen, grobmaschigen Sieb (Abtrennsieb) und zwei darunterliegenden, engmaschigen Sieben (Hilfssiebe) gegossen. Die nicht vorgequollenen Polymergele wurden direkt auf das Abtrennsieb gebracht. Die Abtrennsiebe hatten Maschenweiten von 2000 μm, bzw. 3550 μm, bzw. 5000 μm. Die Hilfssiebe besaßen Maschenweiten von 630 μm und 400 μm. Der Durchmesser betrug jeweils 20 cm. Die Hilfssiebe dienten der Bestimmung des Anteils an Polymergel, welcher die Abtrennsiebe passierte.

Die Polymergele wurden jeweils gleichmäßig auf dem Abtrennsieb verteilt und dann für 40 Sekunden mit einem Wasserstrahl aus 11 cm Höhe (7,5 Liter demineralisiertes Wasser pro Minute; 8 mm Schlauchinnendurchmesser) beaufschlagt. Der Wasserstrahl traf das Polymergel über die 40 Sekunden auf dem Sieb immer in gleichmäßigen Bewegungen, um die gesamte Fläche vergleichbar zu überstreichen. Anschließend wurden die Siebe zum Abtropfen des Wassers für 5 Minuten auf einen Eimer gestellt, dann zur Quantifizierung für 2 Stunden in einem Umlufttrockenschrank bei 135°C vor- und weitere ca.16 Stunden im Vakuum endgetrocknet. Die Masse des getrockneten Polymergels auf dem Abtrennsieb (Ausbeute) und die auf den beiden Hilfssieben (Verlust) wurde bestimmt und in Tabelle 1 prozentual dargestellt.

Die Ergebnisse sind in der Tabelle 1 zusammengefasst.

Tab. 1: Ausbeute an Polymergel nach der Abtrennung

| Polymergel | DN [mol-%] | FG [Gew.-%] | FSC$_{DIW}$ [g/g] | Abtrennsieb [μm] | Quellzeit [min] | Ausbeute [%] | Verlust [%] |
|---|---|---|---|---|---|---|---|
| Bsp. 1 | 75 | 35,0 | 162 | 2000 | 0 | 99,4 | 0,6 |
| | | | | | 3 | 99,1 | 0,9 |
| | | | | | 30 | 94,8 | 5,2 |
| | | | | | 60 | 92,8 | 7,2 |
| Bsp. 2 | 65 | 35,5 | 195 | 3550 | 0 | 100 | 0,0 |
| | | | | | 3 | 100 | 0,0 |
| | | | | | 30 | 94,3 | 5,7 |
| | | | | | 60 | 88,9 | 11,1 |
| Bsp. 2 | 65 | 35,5 | 195 | 5000 | 0 | 95,9 | 4,1 |
| | | | | | 3 | 88,6 | 11,4 |
| | | | | | 30 | 80,0 | 20,0 |
| | | | | | 60 | 75,8 | 24,2 |
| DN Netralisationsgrad FG Feststoffgehalt | | | | | | | |

Die Beispiele zeigen, dass das Polymergel mit zunehmender Verweilzeit im Spülwasser nur noch unvollständig abgetrennt werden kann.

**Patentansprüche**

**1.** Verfahren zur Herstellung von Superabsorberpartikeln durch Polymerisation einer Monomerlösung, enthaltend

a) mindestens ein ethylenisch ungesättigtes, säuregruppentragendes Monomer, das zu 50 bis 85 mol-% neutralisiert ist,

b) mindestens einen Vernetzer,

c) mindestens einen Initiator,

d) optional ein oder mehrere mit den unter a) genannten Monomeren copolymerisierbare ethylenisch ungesättigte Monomere und

e) optional ein oder mehrere wasserlösliche Polymere,

umfassend die Schritte

i) kontinuierliche Polymerisation der Monomerlösung auf dem umlaufenden Band eines Bandreaktors,

ii) Zerkleinerung des in Schritt i) erhaltenen Polymergels,

iii) Spülen des auf der Unterseite des Bandreaktors anfallenden Polymergels mit Wasser in eine Abtrenneinheit,

iv) Abtrennung des Polymergels vom Wasser in der Abtrenneinheit und

v) Rückführung des in Schritt iv) abgetrennten Polymergels in Schritt ii),

wobei das Polymergel eine Quellkapazität in Wasser von mindestens 100 g/g aufweist, die Verweilzeit zwischen Schritt iii) und Schritt iv) weniger als 20 Minuten beträgt und die Abtrenneinheit in Schritt iv) Öffnungen mit einer lichten Weite von mindestens 1 mm aufweist.

2. Verfahren nach Anspruch 1, **dadurch gekennzeichnet, dass** die Verweilzeit zwischen Schritt iii) und Schritt iv) weniger als 15 Minuten beträgt.

3. Verfahren nach Anspruch 1 oder 2, **dadurch gekennzeichnet, dass** die Verweilzeit zwischen Schritt iii) und Schritt iv) weniger als 10 Minuten beträgt.

4. Verfahren nach einem der Ansprüche 1 bis 3, **dadurch gekennzeichnet, dass** die Verweilzeit zwischen Schritt iii) und Schritt iv) weniger als 5 Minuten beträgt.

5. Verfahren nach einem der Ansprüche 1 bis 4, **dadurch gekennzeichnet, dass** das auf der Unterseite des Bandreaktors anfallende Polymergel in einer Wanne aufgefangen wird.

6. Verfahren nach einem der Ansprüche 1 bis 5, **dadurch gekennzeichnet, dass** die Abtrenneinheit in Schritt iv) Öffnungen mit einer lichten Weite von mindestens 1,5 mm aufweist.

7. Verfahren nach einem der Ansprüche 1 bis 6, **dadurch gekennzeichnet, dass** die Abtrenneinheit in Schritt iv) Öffnungen mit einer lichten Weite von mindestens 2 mm aufweist.

8. Verfahren nach einem der Ansprüche 1 bis 7, **dadurch gekennzeichnet, dass** die Abtrenneinheit in Schritt iv) Öffnungen mit einer lichten Weite von mindestens 3 mm aufweist.

9. Verfahren nach einem der Ansprüche 1 bis 8, **dadurch gekennzeichnet, dass** die Öffnungen der Abtrenneinheit in Schritt iv) aus den Maschen eines Siebes bestehen.

10. Verfahren nach einem der Ansprüche 1 bis 8, **dadurch gekennzeichnet, dass** die Öffnungen der Abtrenneinheit in Schritt iv) die Form von Schlitzen aufweisen.

11. Verfahren nach einem der Ansprüche 1 bis 10, **dadurch gekennzeichnet, dass** Abtrenneinheit eine liegende, rotierende Trommel ist und die Trommel vom Eingang zum Ausgang geneigt ist.

12. Verfahren nach Anspruch 11, **dadurch gekennzeichnet, dass** die Trommel um mindestens 10° gegenüber der Horizontalen geneigt.

13. Verfahren nach Anspruch 11 oder 12, **dadurch gekennzeichnet, dass** die Trommel um mindestens 20° gegenüber der Horizontalen geneigt.

14. Verfahren nach einem der Ansprüche 11 bis 13, **dadurch gekennzeichnet, dass** die Trommel um mindestens 30° gegenüber der Horizontalen geneigt.

**15.** Verfahren nach einem der Ansprüche 1 bis 14, **dadurch gekennzeichnet, dass** in Schritt v) erhaltene Wasser in das Verfahren in Schritt iii) rückgeführt wird.

**Claims**

**1.** A process for producing superabsorbent particles by polymerizing a monomer solution comprising

a) at least one ethylenically unsaturated monomer which bears acid groups and has been neutralized to an extent of 50 to 85 mol%,
b) at least one crosslinker,
c) at least one initiator,
d) optionally one or more ethylenically unsaturated monomers copolymerizable with the monomers mentioned under a) and
e) optionally one or more water-soluble polymers, comprising the steps of

i) continuously polymerizing the monomer solution on the continuous belt of a belt reactor,
ii) comminuting the polymer gel obtained in step i),
iii) rinsing the polymer gel obtained on the underside of the belt reactor into a separation unit with water,
iv) separating the polymer gel from the water in the separation unit and
v) recycling the polymer gel separated off in step iv) into step ii),

where the polymer gel has a swelling capacity in water of at least 100 g/g, the dwell time between step iii) and step iv) is less than 20 minutes, and the separation unit in step iv) has openings with a clear width of at least 1 mm.

**2.** The process according to claim 1, wherein the dwell time between step iii) and step iv) is less than 15 minutes.

**3.** The process according to claim 1 or 2, wherein the dwell time between step iii) and step iv) is less than 10 minutes.

**4.** The process according to any of claims 1 to 3, wherein the dwell time between step iii) and step iv) is less than 5 minutes.

**5.** The process according to any of claims 1 to 4, wherein the polymer gel obtained on the underside of the belt reactor is collected in a tank.

**6.** The process according to any of claims 1 to 5, wherein the separation unit in step iv) has openings having a clear width of at least 1.5 mm.

**7.** The process according to any of claims 1 to 6, wherein the separation unit in step iv) has openings having a clear width of at least 2 mm.

**8.** The process according to any of claims 1 to 7, wherein the separation unit in step iv) has openings having a clear width of at least 3 mm.

**9.** The process according to any of claims 1 to 8, wherein the openings in the separation unit in step iv) consist of the meshes of a screen.

**10.** The process according to any of claims 1 to 8, wherein the openings in the separation unit in step iv) take the form of slots.

**11.** The process according to any of claims 1 to 10, wherein the separation unit is a horizontal rotating drum, and the drum is inclined from the inlet to the outlet.

**12.** The process according to claim 11, wherein the drum is inclined by at least 10° relative to the horizontal.

**13.** The process according to claim 11 or 12, wherein the drum is inclined by at least 20° relative to the horizontal.

**14.** The process according to any of claims 11 to 13, wherein the drum is inclined by at least 30° relative to the horizontal.

**15.** The process according to any of claims 1 to 14, wherein water obtained in step v) is recycled into the process in step iii).

**Revendications**

**1.** Procédé de fabrication de particules superabsorbantes par polymérisation d'une solution de monomères, contenant

a) au moins un monomère à insaturation éthylénique portant des groupes acides, qui est neutralisé à 50 à 85 % en moles,
b) au moins un agent de réticulation,
c) au moins un amorceur,
d) éventuellement un ou plusieurs monomères à insaturation éthylénique copolymérisables avec les monomères mentionnés en a) et
e) éventuellement un ou plusieurs polymères solubles dans l'eau,

comprenant les étapes suivantes :

i) polymérisation continue de la solution de monomères sur la bande mobile d'un réacteur à bande,
ii) broyage du gel polymère obtenu dans l'étape i),
iii) rinçage à l'eau, dans une unité de séparation, du gel polymère arrivant sur la face inférieure du réacteur à bande,
iv) séparation du gel polymère de l'eau dans l'unité de séparation et
v) renvoi à l'étape ii) du gel polymère séparé dans l'étape iv),

le gel polymère présentant une capacité de gonflement dans l'eau d'au moins 100 g/g, le temps de séjour entre l'étape iii) et l'étape iv) étant inférieur à 20 minutes, et l'unité de séparation de l'étape iv) comportant des ouvertures ayant une largeur de passage d'au moins 1 mm.

**2.** Procédé selon la revendication 1, **caractérisé en ce que** le temps de séjour entre l'étape iii) et l'étape iv) est inférieur à 15 minutes.

**3.** Procédé selon la revendication 1 ou 2, **caractérisé en ce que** le temps de séjour entre l'étape iii) et l'étape iv) est inférieur à 10 minutes.

**4.** Procédé selon l'une des revendications 1 à 3, **caractérisé en ce que** le temps de séjour entre l'étape iii) et l'étape iv) est inférieur à 5 minutes.

**5.** Procédé selon l'une des revendications 1 à 4, **caractérisé en ce que** le gel polymère arrivant sur la face inférieure du réacteur à bande est repris dans une cuve.

**6.** Procédé selon l'une des revendications 1 à 5, **caractérisé en ce que** l'unité de séparation de l'étape iv) comprend des ouvertures ayant une largeur de passage d'au moins 1,5 mm.

**7.** Procédé selon l'une des revendications 1 à 6, **caractérisé en ce que** l'unité de séparation de l'étape iv) comprend des ouvertures ayant une largeur de passage d'au moins 2 mm.

**8.** Procédé selon l'une des revendications 1 à 7, **caractérisé en ce que** l'unité de séparation de l'étape iv) comprend des ouvertures ayant une largeur de passage d'au moins 3 mm.

**9.** Procédé selon l'une des revendications 1 à 8, **caractérisé en ce que** les ouvertures de l'unité de séparation de l'étape iv) sont constituées des mailles d'un tamis.

**10.** Procédé selon l'une des revendications 1 à 8, **caractérisé en ce que** les ouvertures de l'unité de séparation de l'étape iv) ont la forme de fentes.

**11.** Procédé selon l'une des revendications 1 à 10, **caractérisé en ce que** l'unité de séparation est un tambour tournant horizontal, le tambour étant incliné de l'entrée vers la sortie.

**12.** Procédé selon la revendication 11, **caractérisé en ce que** le tambour est incliné d'au moins 10° par rapport à l'horizontale.

**13.** Procédé selon la revendication 11 ou 12, **caractérisé en ce que** le tambour est incliné d'au moins 20° par rapport à l'horizontale.

**14.** Procédé selon l'une des revendications 11 à 13, **caractérisé en ce que** le tambour est incliné d'au moins 30° par rapport à l'horizontale.

**15.** Procédé selon l'une des revendications 1 à 14, **caractérisé en ce que** l'eau obtenue dans l'étape v) est renvoyée dans le procédé de l'étape iii).

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3825366 A1
- US 6241928 B
- EP 2371869 A1
- WO 02055469 A1
- WO 03078378 A1
- WO 2004035514 A1
- EP 0530438 A1
- EP 0547847 A1
- EP 0559476 A1
- EP 0632068 A1
- WO 9321237 A1
- WO 03104299 A1
- WO 03104300 A1
- WO 03104301 A1
- DE 10331450 A1
- DE 10331456 A1
- DE 10355401 A1
- DE 19543368 A1
- DE 19646484 A1
- WO 9015830 A1
- WO 02032962 A2
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 03031482 A1
- DE 3713601 A1
- EP 0703265 B1
- US 5840321 A

**In der Beschreibung aufgeführte Nicht-Patentliteratur**

- Modern Superabsorbent Polymer Technology. **F.L. BUCHHOLZ ; A.T. GRAHAM.** Monographie. Wiley-VCH, 1998, 71-103
- Standard Test Methods for the Nonwovens Industry. Worldwide Strategie Partners. EDANA, 2005